(19) Europäisches Patentamt
European Patent Office
Office européen des brevets

(11) **EP 4 728 992 A1**

(12) **EUROPEAN PATENT APPLICATION**

(43) Date of publication:
**22.04.2026 Bulletin 2026/17**

(21) Application number: **24206607.4**

(22) Date of filing: **15.10.2024**

(51) International Patent Classification (IPC):
*A61B 8/08* (2006.01)    *A61B 8/00* (2006.01)
*G06T 5/00* (2024.01)    *G06T 5/70* (2024.01)

(52) Cooperative Patent Classification (CPC):
**A61B 8/461; A61B 8/0866; A61B 8/467;**
**A61B 8/469;** A61B 8/5269

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **KOTHAWALA, Aliarshad Aameer**
**Eindhoven (NL)**
• **SHETTIGAR, Srikanth**
**Eindhoven (NL)**
• **VELUMURGAN, Mylavan**
**5656AG Eindhoven (NL)**

• **FIRTION, Celine**
**Eindhoven (NL)**
• **SETH, Subhendu**
**Eindhoven (NL)**
• **VAJINEPALLI, Pallavi**
**Eindhoven (NL)**
• **VAYYETI, Anudeep**
**Eindhoven (NL)**
• **SUBASH CHANDRAN, Dheepak**
**Eindhoven (NL)**
• **AWASTHI, Manish**
**Eindhoven (NL)**
• **NELLUR PRAKASH, Sindhu Priyadarshini**
**Eindhoven (NL)**

(74) Representative: **Philips Intellectual Property &**
**Standards**
**High Tech Campus 52**
**5656 AG Eindhoven (NL)**

(54) **PROCESSING OF A DIGITAL ULTRASOUND IMAGE**

(57)    A mechanism for controlling image processing performed on a portion of a digital ultrasound image. One or more display parameters for display of the portion of the digital ultrasound image are used to modify or define one or more parameters of at least one spatial filtering function applied to the portion of the digital ultrasound image.

FIG. 2

EP 4 728 992 A1

**Description**

FIELD OF THE INVENTION

The present invention relates to the field of ultrasound imaging, and in particular, to the processing of a digital ultrasound image.

BACKGROUND OF THE INVENTION

In the field of medicine, there is an increasing reliance on medical imaging procedures to assess the condition of a medical subject and/or to diagnose any pathology of the medical subject. One particularly widespread medical imaging technique is ultrasound imaging, which produces digital ultrasound images for assessment. This imaging modality plays an important role in various medical disciplines, including Obstetrics and Gynecology, General Imaging, and Cardiology.

It is common for a digital ultrasound image to undergo some image processing procedures, such as noise filtering, contrast enhancement and/or sharpening. The aim of such image processing procedures is to improve the appearance of the digital ultrasound image when displayed, to improve an ease of performing assessment and/or to reduce a risk of important features or element being overlooked.

There is therefore an ongoing demand to improve the processing of digital ultrasound images.

SUMMARY OF THE INVENTION

The invention is defined by the claims.

There is herein proposed a computer-implemented method for processing a digital ultrasound image.

The computer-implemented method comprises: receiving an indicator of at least one display parameter for a portion of the digital ultrasound image, being a digital ultrasound image portion; and processing the digital ultrasound image portion using one or more spatial filtering functions to produce a filtered digital ultrasound image portion. One or more parameters of a set of the one or more spatial filtering functions are responsive to the indicator of the at least one display parameter.

The present disclosure provides a mechanism for performing image processing that is at least partially dependent upon one or more display properties for displaying the image. In particular, at least one spatial filtering function for processing a digital ultrasound image is dependent upon (i.e., changes responsive to) at least one display parameter for the digital ultrasound image.

The proposed method provides dynamic adaptation of image processing based on display parameters, allowing for improved visualization and image quality of specific regions of interest in ultrasound images when displayed. The responsive nature of the spatial filtering functions aims to enhance the clarity and detail of the displayed image portion.

In some embodiments, the set of the one or more spatial filtering functions are configured to process the digital ultrasound image by convolving the digital ultrasound image with a respective kernel; and one or more parameters of each respective kernel are responsive to the indicator of the at least one display parameter.

The use of convolution with adaptive kernels allows for precise and localized image enhancement, tailoring the processing to the specific characteristics of the displayed region.

In some embodiments, the at least one display parameter comprises a desired zoom level indicating a desired magnification of the digital ultrasound image portion when displayed. Due to restricted display space, the desired zoom level will at least partially define or influence a size of the digital ultrasound image portion with respect to the (overall) digital ultrasound image.

Incorporating zoom level into the processing parameters allows for adaptive enhancement that adapts or improves image quality across different magnification levels.

In some embodiments, the at least one display property comprises a desired location of the digital ultrasound image portion within the digital ultrasound image.

This embodiment recognizes that different areas of the digital ultrasound image will have different image quality characteristics, due to the inherent nature of ultrasound imaging. In particular, parts of an ultrasound image that represent regions distanced from the source of ultrasound waves will typically have a lower resolution than regions closer to the source of ultrasound waves (due at least to natural divergence and attenuation of ultrasound waves).

By considering the location of the displayed portion within the overall image, the image processing can thereby be improved optimized to account for these varying characteristics. This location-aware processing can thereby improve the visibility of structures at different depths or in areas with varying acoustic properties.

In some embodiments, different parts of the digital ultrasound image represent different distances from an ultrasound transducer used to generate the digital ultrasound image; and the indicator of the at least one display property identifies a region of the digital ultrasound image in which the desired location lies. These embodiments allow for depth-specific image enhancement, accounting for the varying characteristics of ultrasound signals at different distances from the transducer.

In some embodiments, the indicator is a user-defined indicator. Allowing user-defined indicators enables customization of the image processing and/or any subsequent display to suit individual preferences or specific diagnostic requirements.

In some embodiments, the computer-implemented method further comprises receiving, via a user input interface, the user-defined indicator of the at least one display parameter. The integration of a user input interface for defining display parameters provides an intuitive and interactive way for users to control image processing.

In some embodiments, the set of the one or more spatial filtering functions comprises a sharpening function configured to sharpen the digital ultrasound image portion. The inclusion of a sharpening function allows for enhanced edge definition and detail visibility in the processed image portion. It has been herein recognized that the display parameter(s) for a portion of a digital ultrasound image would affect the most advantageous sharpening procedure to be performed on said portion.

In some examples, the one or more parameters of the sharpening function are configured to increase the intensity of sharpening performed on the image data for increasing desired zoom levels indicated by the indicator.

In some examples, the one or more parameters of the sharpening function are configured to increase the intensity of sharpening performed on the image data for increasing proximity of the desired location of the digital ultrasound image portion to a part of the digital ultrasound image representing a shortest distance to an ultrasound transducer.

In some embodiments, the set of the one or more spatial filtering functions comprises a contrast enhancement function configured to enhance a contrast of the digital ultrasound image portion.

Contrast enhancement improves the visibility of subtle differences in echogenicity, aiding in the detection of small parts of the imaged region (e.g., lesions or subtle tissue changes). This feature can enhance the overall readability of the ultrasound image, potentially leading to more confident and accurate interpretations.

In some embodiments, the computer-implemented method further comprises displaying the filtered digital ultrasound image portion at an output user interface.

There is also herein proposed a computer program product comprising computer program code means which, when executed on a computing device having a processing system, cause the processing system to perform all of the steps of any herein proposed method.

There is also herein proposed a processing system for processing a digital ultrasound image, the processing system being configured to: receive an indicator of at least one display parameter defining, as a digital ultrasound image portion, a portion of the digital ultrasound image for display; and process the digital ultrasound image portion using one or more spatial filtering functions to produce a filtered digital ultrasound image portion, wherein one or more parameters of a set of the one or more spatial filtering functions are responsive to the indicator of the at least one display parameter.

There is also herein proposed an interface system comprising: the processing system and an output user interface configured to display the digital ultrasound image portion.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a system in which embodiments may be employed;
Fig. 2 is a flowchart illustrating a proposed method;
Fig. 3 illustrates a workflow resulting from performing the proposed method; and
Fig. 4 illustrates a proposed processing system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for controlling image processing performed on a portion of a digital ultrasound image. One or more display parameters for display of the portion of the digital ultrasound image are used to modify or define one or more parameters of at least one spatial filtering function applied to the portion of the digital ultrasound image.

The present disclosure thereby provides a technique for dynamically adapting spatial filtering functions in the processing of digital ultrasound images based on display parameters. This approach allows for tailored image enhancement that is able to improve the visibility and (where relevant) diagnostic value of specific regions or portions of interest within the ultrasound image when displayed.

In the context of the present disclosure, the term "display parameter" refers to any characteristic or setting that influences how a digital ultrasound image portion is presented or rendered for viewing. Example display parameters may include, but are not limited to, factors such as zoom/magnification level, position coordinates within the overall image, region of interest selection, or any other attribute that affects the visual representation of a specific part of an image.

In the context of the present disclosure, the term "spatial filtering function" refers to an image processing operation that modifies pixel/voxel values of an image based on the values of neighboring pixels/voxels. These functions may be implemented through techniques such as convolution with a kernel, mathematical transformations, or other computational methods that consider the spatial relationships between pixels/voxels in the image.

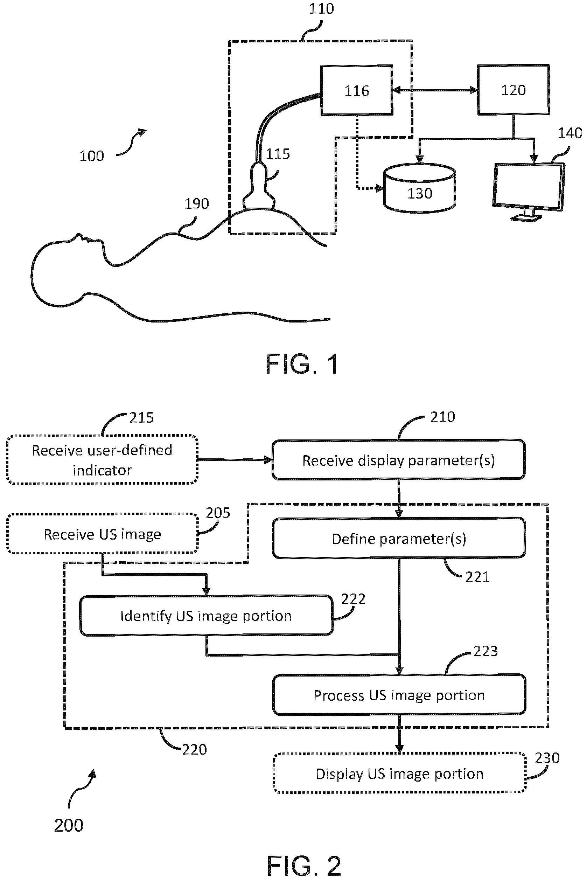

Fig. 1 illustrates a system 100 in which proposed embodiments may be employed. The system 100 comprises an ultrasound imaging system 110, a processing system 120 and an output user interface 140. The processing system 120 and the output user interface 140 together form an interface system.

The ultrasound imaging system 110 is configured to generate a digital ultrasound image 115. The digital ultrasound image 115 provides a data or digital representation of an imaged region, e.g., of a medical subject 190. The digital ultrasound image 115 may, for instance, be a two-dimensional (2D) digital image, a three-dimensional (3D) digital image or a digital image defining more than three dimensions.

The digital ultrasound image 115 may be represented as a grid of discrete elements called pixels (for 2D images) or voxels (for 3D images). Each pixel or voxel corresponds to a specific location in the imaged region and contains information about the intensity or other properties of one or more received ultrasound signals at/from that location.

More particularly, the digital ultrasound image 115 comprises a plurality of discrete image elements (i.e., pixels or voxels). Each image element represents a different sub-region of the imaged region.

The general function of an ultrasound imaging system 110 to produce a digital ultrasound image is well known in the art, and is not described in detail for the sake of conciseness.

Nonetheless, it is noted that in a typical ultrasound imaging system, capture of the digital ultrasound image is performed using an ultrasound probe 112 of the ultrasound imaging system 110 in communication with an imaging system processor 113. The ultrasound probe 112 typically comprises at least one transducer array with multiple transducer elements designed for both transmitting and receiving ultrasound waves. During operation, each transducer element can transmit ultrasound waves in the form of at least one transmit impulse with a specific pulse duration. The transmitted waves propagate through the structure being examined, and as they encounter interfaces between different structures or elements, some of the waves are reflected back towards the probe. Each transducer element then receives these reflected ultrasound waves and converts them into electrical signals.

The received signals are then processed by the imaging system processor 113 to produce the digital ultrasound image. In particular, the imaging system processor 113 performs a number of processes on the received signals, including beamforming (which involves combining signals from multiple elements to focus the ultrasound beam) and signal processing (e.g., to enhance the quality of the image). The processed signals are then used to construct the digital ultrasound image, e.g., by converting the time-domain signals into spatial information defining the digital ultrasound image.

The resulting digital ultrasound image thereby provides a data representation of the internal structures of the imaged region.

The digital ultrasound image 115 is passed to the processing system 120. The processing system 120 is configured to perform one or more image processing procedures on the digital ultrasound image 115, such as noise filtering, contrast enhancement and/or sharpening.

One approach to performing an image processing procedure is to process the digital ultrasound image using a spatial filtering function. In general, a spatial filtering function operates by modifying the value(s) of each pixel/voxel using the values of only neighboring pixel/voxels to said pixel/voxel.

For each (modified) pixel/voxel, the neighboring pixels/voxels may include only a restricted number of pixels/voxels in the vicinity of said pixel/voxel (i.e., and not all pixels/voxels of the ultrasound medical image). For instance, for each pixel, the neighboring pixels/voxels may include only those pixels/voxels of the digital ultrasound image lying in a predetermined grid that includes said pixel/voxel.

For instance, spatial filtering may be performed by convolving the digital ultrasound image with a kernel (sometimes labelled a mask). The kernel is a small matrix of numerical coefficients that is systematically moved across the digital ultrasound image. At each position, the kernel is multiplied element-wise with the corresponding image pixels/voxels, and the results are summed to produce a new pixel/voxel value in the filtered image. The new pixel/voxel value replaces the value of a predetermined (e.g., central) pixel/voxel with respect to the kernel. The kernel is smaller than the digital ultrasound image.

A more complete description of how to perform convolution using one or more kernels, in the context of image processing, is provided by Kim, Sung, and Riley Casper. "Applications of convolution in image processing with MATLAB." University of Washington (2013): 1-20 or Roerdink, Jos BTM. "An introduction to digital image processing." Digital Image Analysis of Microbes: Imaging, Morphometry, Fluorometry and Motility Techniques and Applications (1998): 1.

A wide variety of spatial filtering functions are known in the art, such as smoothing filters, sharpening filters, contrast enhancement filtered, edge detection filters and so on.

The processing system 120 may also be configured to control the output user interface 140 to provide a visual representation (i.e., display) of the (processed) digital ultrasound image. This provides a user of the system 100 with a visual representation of the internal structures of the imaged region, which is particularly useful in the field of medicine for assessing and/or analyzing the condition of the imaged region (and corresponding medical subject 190).

The output user interface 140 may therefore comprise a screen, monitor or other display device configured to controllably provides a visual representation of a digital image, such as the digital ultrasound image.

The system 100 may further comprise a database 130 or memory unit. The ultrasound imaging system 110 may be configured to store the digital ultrasound image 115 in the database 130. Once stored in the database 130, the digital ultrasound image may be retrieved (e.g., at a later time) by the processing system 130.

In some examples, the database 130 may be a PACS database that allows for the confidential storage and retrieval of medical images and imaging data between different medical devices and different medical institutions.

The processing system 120 may retrieve the digital ultrasound image 115 from the database or directly from the ultrasound imaging system 110.

The present disclosure proposes a technique for performing image processing of the digital ultrasound image 115 that may be employed by the processing system 120. In particular, the present disclosure proposes a technique that adapts one or parameters of the image processing responsive to at least one display parameter for (a portion of) the digital ultrasound image.

This provides a mechanism for dynamically adjusting image processing parameters based on how the ultrasound image or a portion thereof will be displayed.

Fig. 2 is a flowchart illustrating a proposed (computer-implemented) method 200 for processing a digital ultrasound image. The method 200 may, for instance, be performed by the processing system 120 (Fig. 1).

The method 200 may comprise a step 205 of receiving the digital ultrasound image ("US image"). As previously explained, this may be performed by retrieving the digital ultrasound image from a database or from an ultrasound imaging system.

The method 200 comprises a step 210 of receiving an indicator of at least one display parameter for a portion of the digital ultrasound image (i.e., a digital ultrasound image portion). Each display parameter defines a characteristic or setting that influences how the portion of the digital ultrasound image is presented or rendered for viewing. It will be appreciated that the portion of the digital ultrasound image may effectively represent a region of interest of the digital ultrasound image.

The digital ultrasound image portion may comprise all of the digital ultrasound image or, preferably, only a part (i.e., not all) of the digital ultrasound image.

In particular, each display parameter may be responsive to the bounds of the portion of the digital ultrasound image. More specifically, each display parameter may be a parameter that (at least partially) defines the size and/or position of the digital ultrasound image portion.

For instance, the display parameter(s) may include a zoom/magnification level for the display of the digital ultrasound image. This defines or influences the size of the portion of the digital ultrasound image. More particularly, due to inherent restrictions on the size of a display space, the desired zoom level will at least partially define or influence a size of the digital ultrasound image portion with respect to the (overall) digital ultrasound image

As used herein, the term "zoom/magnification level" refers to a display parameter that determines the degree of enlargement or reduction applied to the digital ultrasound image portion when presented on a display. The zoom/magnification level may be expressed as a numerical factor (e.g., 2x, 4x) or a percentage (e.g., 200%, 400%) relative to the original image size.

The skilled person will appreciate how, as the zoom level increases, the portion may represent a smaller area of the original image, for later showing at a larger scale. For example, at a 2x zoom level, the digital ultrasound image portion may cover only 50% of the original image area, (e.g., but be later displayed at twice the size). At a 4x zoom level, the portion may cover only 25% of the original image area (e.g., but be later displayed at four times the size).

The display parameter(s) may include a desired location of the digital ultrasound image portion within the digital ultrasound image.

As used herein, the term "desired location" refers to a specific position or area within the digital ultrasound image that is selected or specified as the digital ultrasound image portion. This location may be defined by coordinates or other spatial indicators within the overall image space.

Other examples of suitable display parameters include a desired brightness for a display of the digital ultrasound image portion and/or a desired contrast for the display of the digital ultrasound image portion.

In some instances, the indicator (received in step 210) is a user-defined indicator. Accordingly, the method 200 may further comprise a step 215 of receiving, via a user input interface, the user-defined indicator of the at least one display parameter.

The user input interface for receiving the user-defined indicator may take various forms. In some cases, it may be a graphical user interface (GUI) displayed on a screen (or other display device), allowing the user to interact with the digital ultrasound image directly. This GUI may include tools for selecting regions of interest (e.g., defining the desired location and zoom level simultaneously), adjusting zoom levels, or specifying coordinates for the desired location.

In some cases, the user input interface may include a cursor-based interaction system that allows the user to manipulate a cursor to identify a desired region of interest and/or desired location within the digital ultrasound image. This cursor may be controlled using various input devices such as a mouse, trackpad, touchscreen, or keyboard.

The user may move the cursor over a display or visual representation of the digital ultrasound image to select a specific area of interest. As the cursor moves, the system may provide real-time feedback, such as highlighting the area under the cursor or displaying coordinates of the current cursor position.

The cursor-based interface may also and/or otherwise allow the user to interact with zoom controls, enabling them to click on specific areas to zoom in or use a scroll wheel to adjust the zoom level dynamically. This interaction between cursor movement and zoom functionality may provide an intuitive way for users to explore and select regions of interest at various scales within the digital ultrasound image.

The method 200 also comprises a step 220 of processing the digital ultrasound image portion using one or more spatial filtering functions to produce a filtered digital ultrasound image portion.

Examples of spatial filtering functions have been previously exemplified, and a wide variety of spatial filtering functions are known in the art, such as smoothing filters, sharpening filters, contrast enhancement filtered, edge detection filters and so on.

In step 220, one or more parameters of a set of the one or more spatial filtering functions are responsive to the indicator of the at least one display parameter.

It will be appreciated that, although possible, not all of the spatial filtering functions used in processing the digital ultrasound image portion need to be responsive to the display parameter(s). Rather, step 220 may employ a combination of adaptive and non-adaptive spatial filtering functions to achieve the desired image processing results.

For example, some spatial filtering functions may have fixed parameters that remain constant regardless of the display parameter(s), while others may have one or more parameters that adapt based on the indicator of the at least one display parameter. This approach allows for a balance between consistent baseline processing and targeted enhancements based on display conditions.

In one variant, step 220 comprises processing only the digital ultrasound image portion (i.e., and no other portion of the digital ultrasound image) using the one or more spatial filtering functions. This advantageously reduces computational resources required for processing the digital ultrasound image, as only the portion of interest is processed. This leads to faster processing times and improved efficiency, especially when dealing with large or high-resolution ultrasound images.

In another variant, step 220 may comprise processing the entire digital ultrasound image using the one or more spatial filtering functions. This approach may be useful if more than the digital ultrasound image portion is to be displayed, for improved contextualization of the digital ultrasound image portion to a viewer of the display.

For the purposes of step 220, it will be appreciated that each of the set of one or more spatial filtering functions $f(\cdot)$ is defined by one or more parameters $\theta_i$. By way of example, where a spatial filtering function $f(\cdot)$ is configured to process the digital ultrasound image by convolution with a kernel, then the value of each weight in the kernel may be considered an i-th parameter $\theta_i$ of the spatial filtering function. Other examples of parameters for a spatial filtering function that make use of kernels include: the size of the kernel and/or the shape of the kernel.

Step 220 may be conceptually divided into a first sub-step 221, a second sub-step 222 and a third sub-step 223.

The first sub-step 221 comprises defining the one or more parameters of the set of one or more spatial filtering function(s) using the at least one display parameter, example approaches for which are later described.

The second sub-step 222 comprises identifying the digital ultrasound image portion ("US image portion") using the at least one display parameter. Sub-step 222 may be performed when each display parameter at least partially defines the bounds of the portion of the digital ultrasound image.

By way of example, in cases where the display parameter includes a zoom/magnification level, sub-step 222 may determine the size and boundaries of the digital ultrasound image portion relative to the full image. For instance, a 2x zoom level may result in identifying a portion that covers 50% of the original image area, while a 4x zoom level may identify a portion covering 25% of the original image area.

When the display parameter specifies a desired location for the digital ultrasound image portion, sub-step 222 may use this information to pinpoint the center or starting point of the digital ultrasound image portion within the overall image space. This may involve translating user-provided coordinates or spatial indicators into specific pixel or voxel locations within the digital ultrasound image.

Of course, sub-step 222 may also consider combinations of display parameters. For example, if both zoom level and desired location are provided, the identification process may involve determining both the size and position of the digital ultrasound image portion simultaneously.

The identification process may also consider the resolution and aspect ratio of the display device on which the digital ultrasound image portion will be shown, adjusting the identified portion as needed to improve its presentation.

The third sub-step 223 comprises processing the digital ultrasound image portion using the one or more spatial filtering functions. As previously mentioned, this may be carried out by processing only the digital ultrasound image portion (identified in sub-step 222) or processing the entire digital ultrasound image using the one or more spatial filtering functions.

To perform sub-step 223, each spatial filtering function may be applied in turn, i.e., sequentially. The order in which the filtering functions are applied may depend upon the nature of the filtering functions and/or user preference.

It has been previously mentioned how the display parameter(s) may include a desired location of the digital ultrasound image portion within the digital ultrasound image. In particular, different parts of the digital ultrasound image may represent different distances from an ultrasound transducer used to generate the digital ultrasound image; and the indicator of the at least one display property identifies a part of the digital ultrasound image in which the desired location lies.

In such examples, in step 220, one or more parameters of a set of the one or more spatial filtering functions may be (defined) responsive to the part of the digital ultrasound image in which the desired location lies.

For instance, the different parts of the digital ultrasound image may comprise three separate parts respectively representing near-field, mid-field (e.g., focal zone) and fair-field/Fraunhofer regions. In ultrasound imaging, the near-field, mid-field, and far-field regions refer to different zones of the ultrasound beam as it propagates through an imaged region.

The near-field region is the area closest to the ultrasound transducer(s). The near-field may be characterized by higher signal intensity but potentially lower resolution. The mid-field region, also known as the focal zone, is the area where the ultrasound beam is most focused and is generally considered to provide the best image quality. The far-field region is the area farthest from the transducer(s), in which the ultrasound wave(s) may begin to diverge and lose focus, and may be characterized by decreased signal intensity and reduced resolution due to attenuation and scattering.

In such examples, the spatial filtering functions may be adapted to account for the specific characteristics of each region in the ultrasound image. The near-field, mid-field, and far-field regions of an ultrasound image typically have distinct properties that may benefit from tailored processing approaches.

Thus, step 220 may dynamically adjust the parameter(s) of the set of spatial filtering function(s) based on the specific location of the digital ultrasound image portion within the overall image. For example, if the desired location spans multiple regions, the system may apply a weighted combination of region-specific processing parameters or implement a smooth transition between different parameter sets.

Once the skilled person appreciate the herein proposed concept of modifying a spatial filtering function responsive to one or more display parameters, the skilled person will readily understand a variety of approaches in which this recognition may be exploited, e.g., to define various modifications to spatial filtering function based on one or more display parameters. A number of non-exhaustive examples are hereafter provided for the sake of descriptive understanding. Other examples will be readily apparent to the skilled person.

In some examples, the set of the one or more spatial filtering functions comprises a sharpening function configured to sharpen the digital ultrasound image portion.

The sharpening function enhances edges and details in the digital ultrasound image portion. More particularly, the sharpening function ensures that fine details and structures within the zoomed region are accentuated, contributing to improved visibility and diagnostic precision.

Consider a scenario in which the sharpening function is performed by performing a convolutional process on (at least) the digital ultrasound image portion using a kernel (a "sharpening kernel"), and the display parameter(s) includes a zoom level of the digital ultrasound image portion.

It is recognized that increased digital zoom will naturally create blurring. Accordingly, the sharpening kernel may be modified to attenuate or reduce a change in image quality that results therefrom.

By way of example, as the zoom level increases, the sharpening kernel may be modified to address the apparent blurring that occurs with increased magnification or zoom.

This can be achieved, for instance, by modifying the values of the sharpening kernel to become more aggressive in edge-enhancement, e.g., by increasing the amplitude of the values of the sharpening kernel for increasing zoom levels.

In this way, as the zoom level increases, the sharpening effect may become more pronounced to compensate for potential loss of detail. The sharpening may be controlled by weights or coefficients in a kernel.

By way of illustrative example only, at lower zoom levels a first sharpening kernel $K_1$ may be used:

$$K_1 = \begin{bmatrix} 0 & -1 & 0 \\ -1 & 5 & -1 \\ 0 & -1 & 0 \end{bmatrix} \tag{1}$$

whereas at high zoom levels, a second sharpening kernel $K_2$ may be used:

$$K_2 = \begin{bmatrix} 0 & -2 & 0 \\ -2 & 9 & -2 \\ 0 & -2 & 0 \end{bmatrix} \tag{2}$$

In some examples, the sharpening process may be designed to be directly proportional to the zoom level, e.g., the following kernel $K_3$ may be used:

$$K_3 = \begin{bmatrix} 0 & -1n & 0 \\ -1n & 5n & -1n \\ 0 & -1n & 0 \end{bmatrix} \tag{3}$$

where n is the normalized zoom level (where 1 = no zoom and 2 = 2 times zoom and so on).

In some examples, as the zoom level increases, the size of the sharpening kernel may be reduced. For instance, at high zoom levels the sharpening kernel may comprise values for no fewer than 9 pixels/voxels, whereas at lower zoom levels, the sharpening kernel may comprise values for no fewer than 25 pixels/voxels.

Consider another scenario in which the sharpening function is performed by performing a convolutional process on (at least) the digital ultrasound image portion using a kernel (a "sharpening kernel"), and the display parameter(s) includes a desired location of the digital ultrasound image portion within the digital ultrasound image.

For instance, for locations of the digital ultrasound image that represent an imaged part closer to the ultrasound transducer(s), the sharpening kernel may be designed to be less aggressive in enhancing edges to avoid amplifying noise. Thus, the amplitude of the values of the sharpening kernel may increase for increasing distance (of the location of the digital ultrasound image portion) from a location of the digital ultrasound image representing a closest position to the ultrasound transducer.

In this way, the sharpening function may be configured to apply different levels of sharpening based on the region of the ultrasound image in which the portion of the ultrasound image lies. For instance, the sharpening may be comparatively less intense in a region representing a near field compared to a region representing a far field, with respect to the ultrasound wave propagation along the depth.

This approach is based on the understanding that the near field is often more chaotic and prone to noise. Applying strong gradients when in this region may risk making the resultant image portion noisy and potentially obscuring important details. Conversely, the far field region may be less chaotic, allowing for stronger gradients to sharpen the image portion without introducing significant noise.

As another example, the size of the kernel may be adapted based on the location. For example, larger kernels may be used in the far-field region to capture more context for edge enhancement, while smaller kernels may be sufficient in the near-field region.

Consider another scenario in which the sharpening function is performed by performing a convolutional process on (at least) the digital ultrasound image portion using a kernel (a "sharpening kernel"), and the display parameter(s) includes a desired brightness of the digital ultrasound image portion.

In this scenario, in regions with higher desired brightness, the sharpening kernel may be modified to be less aggressive (i.e., a lower intensity) to avoid over-enhancing already bright areas. Conversely, in regions with lower desired brightness, the sharpening kernel may be adjusted to be more aggressive in enhancing edges and details to improve visibility. This adaptive approach may help maintain a balance between sharpness and overall image brightness, thereby improving the visibility of structures within the digital ultrasound image portion.

The foregoing examples are merely exemplary, and the skilled person would readily understand and be able to define other techniques for modifying one or more parameters of a sharpening function responsive to the display parameter(s).

In some examples, the set of the one or more spatial filtering functions comprises a contrast enhancement function configured to enhance a contrast of the digital ultrasound image portion.

This helps to overall visibility and distinction of structures in the digital ultrasound image portion. More particularly, contrast enhancement improves the differentiation between adjacent pixels/voxels, enhancing the overall clarity of the magnified area and aiding in the identification of subtle anatomical features.

As an example, if the one or more display parameters includes a zoom level, then the contrast enhancement function may adjust its intensity based on the zoom level. For instance, as the zoom level increases, the contrast enhancement may become more pronounced to compensate for potential loss of detail.

As another example, if the one or more display parameters comprises a desired location of the digital ultrasound image portion within the digital ultrasound image, then the contrast enhancement function may adjust its intensity responsive to the desired location. For instance, the contrast enhancement function may increase the intensity of the contrast enhancement for increased distance of the desired location from a location representing the part closest to the ultrasound transducer. This accounts for an understanding that there is ultrasound signal attenuation with increased distance from the transducer, meaning that resolution is decreased with increasing distance, such that aggressive contrast enhancement may help bring out subtle features.

Consider another example where the one or more display parameters includes a brightness parameter, e.g., indicating a desired overall luminance level for the displayed digital ultrasound image portion. In this example, the contrast enhancement function may adjust its intensity inversely proportional to the brightness parameter. Thus, as the desired brightness increases, the contrast enhancement may be reduced to avoid over-saturation of the image. Conversely, for lower brightness settings, the contrast enhancement may be intensified to improve visibility of subtle features.

Consider another example where the one or more display parameters includes a contrast parameter, e.g., indicating a desired overall contrast for the displayed digital ultrasound image portion. In this example, the contrast enhancement function may adjust its intensity based on the contrast parameter. For instance, if the desired contrast is high, the contrast enhancement function may apply a more aggressive enhancement to further accentuate differences between adjacent structures. Conversely, if the desired contrast is low, the contrast enhancement function may apply a more subtle enhancement to avoid over-exaggerating differences in the image.

Where both a sharpening function and a contrast enhancement function are used, the contrast enhancement function may be performed after the sharpening function.

In some examples, the/each spatial filtering function may be designed as a two-dimensional gradient function that takes into account the anisotropic nature of ultrasound imaging, i.e., is an asymmetric spatial filtering function. This approach recognizes that ultrasound images have direction-dependent characteristics, which may be exploited to enhance image quality.

More particularly, it has been recognized that in ultrasound imaging, fine spatial variations are better captured along the direction of ultrasound wave propagation (i.e., have better resolutions). Accordingly, it is herein proposed to make the gradients of a spatial filtering function along the direction of the digital ultrasound image (i.e., an axial direction) corresponding to the direction of ultrasound wave propagation stronger than in a perpendicular direction (i.e., a lateral direction). This ensures that the finer details are more heavily relied upon in the performance of spatial filtering.

This approach takes into account the point spread function of ultrasound, which may be approximated as an ellipse. The major axis of this ellipse may be along the lateral direction, while the minor axis may be along the axial direction. The spatial function(s) may therefore exploit this knowledge to apply gradients in a manner that aligns with the inherent characteristics of ultrasound image formation.

By way of example, the spatial filtering function may be designed to be approximately four times stronger along the axial (beam propagation or depth) direction compared to the lateral direction of the digital ultrasound image.

If the set of one or more spatial filtering functions are performed by convolving the digital ultrasound image with a (respective) kernel, then a two-dimensional gradient function can be readily achieved through appropriate selection of values or coefficients in the kernel, e.g., to more greatly weight coefficients lying in an axial direction compared to those lying in a lateral direction. For digital ultrasound images, it is usually assumed that a direction from an uppermost to lowermost part of the digital ultrasound image (i.e., a top-to-bottom) represents an axial direction, with a side-to-side (e.g., left-to-right or right-to-left) representing the lateral direction.

In one working example, the set of one or more spatial filtering functions comprises a sharpening function that performs a convolution using a sharpening kernel $K_4$, such as:

$$K_4 = \begin{bmatrix} 0 & -4 & 0 \\ -1 & 11 & -1 \\ 0 & -4 & 0 \end{bmatrix} \qquad (4)$$

To take account of different zoom levels and/or position of the digital ultrasound image portion, the following sharpening kernel $K_5$ may (e.g., instead) be used:

$$K_5 = \begin{bmatrix} 0 & -2\mathrm{xn} & 0 \\ -n & 5\mathrm{xn}+1 & -n \\ 0 & -2\mathrm{xn} & 0 \end{bmatrix} \qquad\qquad (5)$$

where n is the normalized zoom level (where 1 represents no zoom and 2 represents a 200% zoom) and x changes responsive to the position of the digital ultrasound image portion in the digital ultrasound image, where lower values of x represent positions closer to a location representing the part closest to the ultrasound transducer. For instance, x may take a value of 1 when the digital ultrasound image portion is in a location close to a part of the digital ultrasound image representing a part proximate to an ultrasound transducer, and a value of 2 otherwise.

It is noted that the example kernels provided in this disclosure are merely illustrative and not limiting. The skilled person may would be readily capable of developing and implementing various kernels for performing one or more spatial filtering functions based on the principles and understanding outlined herein. The specific values, sizes, and structures of the kernels may be defined, e.g., based on the specific display parameters being considered.

For instance, the skilled person will be readily capable of designing kernels that incorporate more complex relationships between zoom levels, image portion position, and spatial filtering. They may also develop kernels that adapt to multiple display parameters simultaneously, such as combining zoom level and brightness considerations in a single kernel.

Furthermore, the skilled person may implement kernels of various sizes and shapes beyond the 3x3 examples provided. Larger kernels may be used to capture more context, while asymmetric kernels may be developed to address specific directional characteristics of the ultrasound image.

The principles described for sharpening kernels may also be applied to other types of spatial filtering functions, such as smoothing or edge detection. The skilled person may adapt these concepts to create a wide range of specialized kernels tailored to specific ultrasound imaging applications or display scenarios.

As previously explained, the indicator received in step 210 may be a user-defined indicator. In this way, the set of spatial filtering function(s) are configured to dynamically adjust based on the user's interactions, reducing a risk of over-enhancement or degradation of image quality for different display parameters.

Of course, the method 200 may further comprise a step 230 of displaying the filtered digital ultrasound image portion at an output user interface. Thus, step 230 may comprise controlling the output user interface to provide a visual representation of the filtered digital ultrasound image portion.

Appropriate techniques for controlling an output user interface to provide a visual representation of a portion of a digital image are well known in the art. For instance, step 230 may comprise converting the digital ultrasound image into a format suitable for display (i.e., display data), which may include color mapping, brightness and/or contrast adjustments, and resolution scaling.

It will be appreciated that the display of the filtered digital ultrasound image portion will be responsive to the display parameter(s). In this way, the display of the ultrasound image may be configured in various ways to accommodate different viewing preferences and diagnostic needs (where relevant).

In some examples, step 230 comprises displaying only the digital ultrasound image portion, effectively providing a focused view of a region of interest. Thus, step 230 may comprise displaying only the digital ultrasound image portion identified in sub-step 222 (if performed). The size of the displayed digital ultrasound image portion may, for instance, be dependent upon a zoom level in the one or more display parameters (if present).

In other examples, the entire digital ultrasound image may be displayed, offering a comprehensive view of the imaged region. This full image display may be beneficial for providing context and allowing for comparison between different regions within the image.

In other examples, step 230 comprises displaying the digital ultrasound image portion along with parts surrounding this portion. This approach may strike a balance between focused examination and contextual awareness.

The choice between these display options is specific to the user-case scenario, and may be dependent upon factors such as user preference and/or the nature of the region being examined. The system may allow for dynamic switching between these display modes, enabling users to adapt their view as needed during the examination process.

It will be appreciated that method 200 may be executed repeatedly, allowing for dynamic and real-time updates to the digital ultrasound image (portion) as the display parameter(s) change. This iterative approach enables repeated or continuous refinement of the image processing based on user interactions or automated adjustments to display settings.

By way of example, the method 200 may be re-executed each time one or more display parameters are updated. This may occur in response to user actions such as zooming, panning, or adjusting brightness and contrast settings for the digital ultrasound image (portion). For example, the user may be able to interact with the displayed image, causing new indicators of the updated display parameters may be received, triggering a new iteration of the method.

Thus, during each iteration, the processing system may receive the updated indicator(s) of the display parameter(s); re-identify the digital ultrasound image portion based on the updated parameters (if necessary); re-process the digital ultrasound image portion using the spatial filtering functions with parameters responsive to the updated display parameter(s); and (if required) update the display of the filtered digital ultrasound image portion.

This continuous processing and updating may provide an image portion that adapts in real-time to changing viewing conditions or user preferences. The spatial filtering functions may thereby be modified reactively and/or "on-the-fly", ensuring that the image quality is continuously adapted for the current or active display settings.

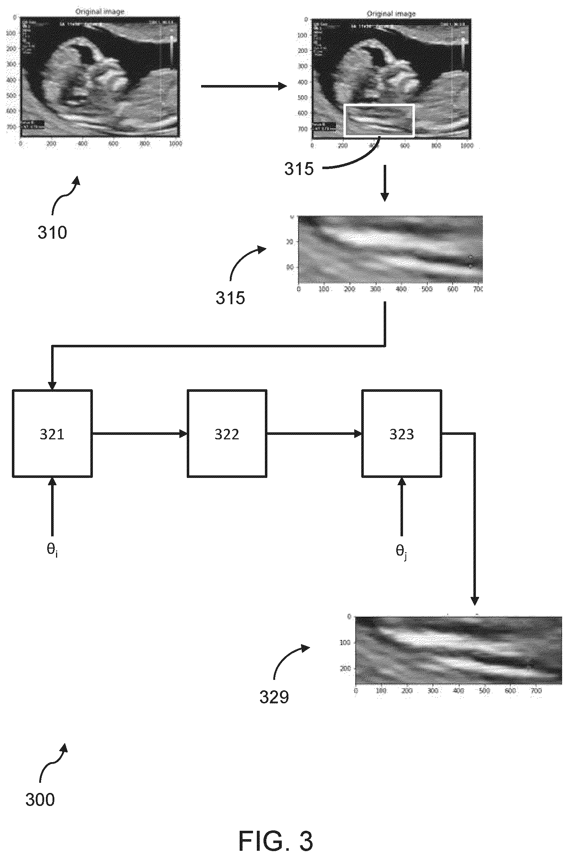

For the sake of illustrative clarity, Fig. 3 illustrates a workflow 300 resulting from the execution of an example of method 200 (Fig. 2).

Initially, a digital ultrasound image 310 is received.

The digital ultrasound image 310 is then processed to identify a portion of the digital ultrasound image (the digital ultrasound image portion 315). This portion represents a region of interest within the digital ultrasound image. The location, size and shape of the digital ultrasound image portion may be user-defined.

The digital ultrasound image portion 315 is then processed using one or more spatial filtering functions 321, 322, 323 to produce a filtered digital ultrasound image portion 335. The one or more spatial filtering functions includes a set of one or more spatial filtering functions each having one or more parameters $\theta_i$, $\theta_j$ that are defined or modified responsive to one or more display parameters 329 for the digital ultrasound image portion.

In the illustrated example, the set 321, 323 of one or more spatial filtering functions includes a sharpening filtering function 321 and a contrast enhancement filtering function 323. The one or more spatial filtering functions further includes a smoothing filtering function 322 (which is not modified by the display parameter(s)).

However, this is merely an exemplary approach, and the skilled person would readily understand or define other suitable filtering functions and whether or not they are modifiable by the display parameter(s).

The filtered digital ultrasound image 329 may then be subsequently displayed.

The skilled person would be readily capable of developing a processing system for carrying out any herein described method. Thus, each step of the flow chart may represent a different action performed by a processing system, and may be performed by a respective module of the processing system.

There is also herein proposed an interface system comprising: the processing system and an output user interface configured to display the digital ultrasound image portion. The output user interface may comprise a screen, monitor or other display device configured to controllably provides a visual representation of a digital image, such as the digital ultrasound image portion.

Some examples outlined above make use of one or more spatial filtering functions that employ a two-dimensional gradient function that takes into account the anisotropic nature of ultrasound image. The skilled person will appreciate that such functions can be employed independently of the proposed approach for modifying the parameter(s) of the spatial filtering function responsive to the indicator of the at least one display parameter.

Thus, there is also proposed a computer-implemented method for processing a digital ultrasound image, the computer-implemented method comprising processing the digital ultrasound image using a spatial filtering function to produce a filtered digital ultrasound image, wherein the spatial filtering functions is a two-dimensional gradient function that applies different weightings for different directions within the digital ultrasound image.

The two-dimensional gradient function used in the spatial filtering function(s) may take into account the inherent anisotropic nature of ultrasound imaging. This approach recognizes that ultrasound images have different characteristics in different directions, particularly along the axial (depth) and lateral dimensions of the ultrasound image. The axial dimension represents a direction in which ultrasound waves propagate away from the ultrasound transducer, whereas the lateral dimension represents a direction perpendicular to the axial dimension.

In some implementations, the two-dimensional gradient function may therefore be configured to apply stronger gradients along the axial direction compared to the lateral direction. This difference in weighting is based on the understanding that ultrasound images typically have better resolution along the axial direction due to the nature of ultrasound wave propagation.

The spatial filtering function may be implemented through various techniques, such as convolution with a specialized kernel. The kernel may be designed to emphasize gradients in the axial direction while applying less emphasis to gradients in the lateral direction. This can be achieved by assigning higher magnitude coefficients to kernel elements along the axial direction.

For example, a simple 3x3 kernel for such a two-dimensional gradient function is illustrated by equation (4) or (5). In these examples, the central column represents the axial direction and has higher magnitude coefficients compared to the outer columns, which represent the lateral directions. This operates under the assumption that a direction from an uppermost to lowermost part of the digital ultrasound image (i.e., a top-to-bottom) represents an axial direction, with a side-to-side (e.g., left-to-right or right-to-left) representing the lateral direction.

The application of such a two-dimensional gradient function may help perform image processing (e.g., sharpening or contrast enhancement) in the ultrasound image while respecting the inherent directional differences in image quality. This may lead to improved visibility of anatomical structures and potentially better diagnostic value of the processed ultrasound images.

It's worth noting that while this approach may be used independently of display parameter-based adaptations, it may also be combined with such techniques to improve image processing based on both image characteristics and display conditions.

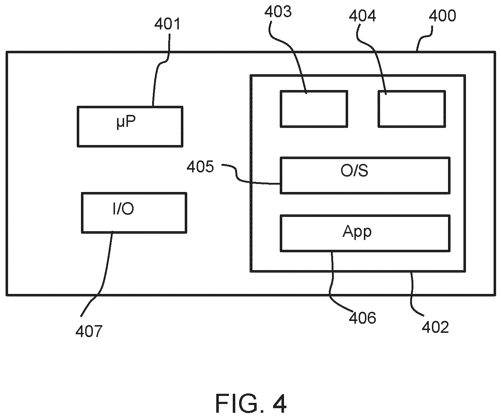

Fig. 4 illustrates an example of a processing system 400 within which one or more parts of an embodiment may be employed.

The processing system 400 provides an example for the processing system 120 (Fig. 1) when configured to perform the method 200 (Fig. 2).

The skilled person will appreciate how the present disclosure also proposes a system 100 comprising the processing system 120, 400 and/or an interface system comprising the processing system 120, 400 and an output user interface 140.

Various operations discussed above may utilize the capabilities of the processing system. For example, one or more parts of a proposed embodiment may be incorporated in any element, module, application, and/or component discussed herein. In this regard, it is to be understood that system functional blocks can run on a single processing system or may be distributed over several processing systems and locations (e.g., connected via internet), such as a cloud-based computing infrastructure.

With continued reference to Fig. 4, the processing system 400 includes, but is not limited to, PCs, workstations, laptops, PDAs, palm devices, servers, storages, and the like. Generally, in terms of hardware architecture, the processing system 400 may include one or more processors 410, memory 420 and one or more I/O devices 430 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 410 is a hardware device for executing software that can be stored in the memory 420. The processor 410 can be virtually any custom made or commercially available processor, a central processing unit (CPU), a digital signal processor (DSP), or an auxiliary processor among several processors associated with the processing system 400, and the processor 410 may be a semiconductor based microprocessor (in the form of a microchip) or a microprocessor.

The memory 420 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 420 may incorporate electronic, magnetic, optical, and/or other types of storage media. Note that the memory 420 can have a distributed architecture, where various components are situated remote from one another, but can be accessed by the processor 410.

The software in the memory 420 may include one or more separate programs, each of which comprises an ordered listing of executable instructions for implementing logical functions. The software in the memory 420 includes a suitable operating system (O/S) 450, compiler 460, source code 470, and one or more applications 480 in accordance with exemplary embodiments. As illustrated, the application 480 comprises numerous functional components for implementing the features and operations of the exemplary embodiments. The application 480 of the processing system 400 may represent various applications, computational units, logic, functional units, processes, operations, virtual entities, and/or modules in accordance with exemplary embodiments, but the application 480 is not meant to be a limitation.

The operating system 450 controls the execution of other computer programs, and provides scheduling, input-output control, file and data management, memory management, and communication control and related services. It is contemplated by the inventors that the application 480 for implementing exemplary embodiments may be applicable on all commercially available operating systems.

Application 480 may be a source program, executable program (object code), script, or any other entity comprising a set of instructions to be performed. When a source program, then the program is usually translated via a compiler (such as the compiler 460), assembler, interpreter, or the like, which may or may not be included within the memory 420, so as to operate properly in connection with the O/S 450. Furthermore, the application 480 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 430 may include input devices such as, for example but not limited to, a mouse, keyboard, scanner, microphone, camera, etc. Furthermore, the I/O devices 430 may also include output devices, for example but not limited to a printer, display, etc. Finally, the I/O devices 430 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the processing system 400 is a PC, workstation, intelligent device or the like, the software in the memory 420 may further include a basic input output system (BIOS) (omitted for simplicity). The BIOS is a set of essential software routines that initialize and test hardware at start-up, start the O/S 450, and support the transfer of data among the hardware devices. The BIOS is stored in some type of read-only-memory, such as ROM, PROM, EPROM, EEPROM or the like, so that the BIOS can be executed when the processing system 400 is activated.

When the processing system 400 is in operation, the processor 410 is configured to execute software stored within the memory 420, to communicate data to and from the memory 420, and to generally control operations of the processing system 400 pursuant to the software. The application 480 and the O/S 450 are read, in whole or in part, by the processor 410, perhaps buffered within the processor 410, and then executed.

When the application 480 is implemented in software it should be noted that the application 480 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 480 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Ordinal numbers (e.g. "first", "second" and so on) have been used purely to distinguish different elements from one another for the sake of clarity, and does not necessarily imply a specific order, importance, relationship, or presence of all numbered elements. Reference to a non-"first" (e.g. "second" or "third") element does not necessitate that a "first" element be present. The skilled person would be capable of relabeling any such elements as appropriate (e.g. relabeling a "second" element as a "first" element if only the second element is present).

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method (200) for processing a digital ultrasound image (310), the computer-implemented method comprising:

    receiving (210) an indicator of at least one display parameter for a portion (315) of the digital ultrasound image, being a digital ultrasound image portion; and

processing (220) the digital ultrasound image portion using one or more spatial filtering functions (321, 322, 323) to produce a filtered digital ultrasound image portion, wherein one or more parameters of a set (321, 323) of the one or more spatial filtering functions are responsive to the indicator of the at least one display parameter.

2. The computer-implemented method of claim 1, wherein:

the set of the one or more spatial filtering functions are configured to process the digital ultrasound image by convolving the digital ultrasound image with a respective kernel; and
one or more parameters $(\theta_i, \theta_j)$ of each respective kernel are responsive to the indicator of the at least one display parameter.

3. The computer-implemented method of claim 1 or 2, wherein the at least one display parameter comprises a desired zoom level indicating a desired magnification of the digital ultrasound image portion when displayed.

4. The computer-implemented method of any one of claims 1 to 3, wherein the at least one display property comprises a desired location of the digital ultrasound image portion within the digital ultrasound image.

5. The computer-implemented method of claim 4, wherein:

different parts of the digital ultrasound image represent different distances from an ultrasound transducer used to generate the digital ultrasound image; and
the indicator of the at least one display property identifies a region of the digital ultrasound image in which the desired location lies.

6. The computer-implemented method of any one of claims 1 to 5, wherein the indicator is a user-defined indicator.

7. The computer-implemented method of claim 6, further comprising receiving (215), via a user input interface, the user-defined indicator of the at least one display parameter.

8. The computer-implemented method of any one of claims 1 to 7, wherein the set of the one or more spatial filtering functions comprises a sharpening function configured to sharpen the digital ultrasound image portion.

9. The computer-implemented method of claim 8, when dependent upon claim 3, wherein one or more parameters of the sharpening function are configured to increase the intensity of sharpening performed on the image data for increasing desired zoom levels indicated by the indicator.

10. The computer-implemented method of claim 8 or 9, when dependent upon claim 4, wherein one or more parameters of the sharpening function are configured to increase the intensity of sharpening performed on the image data for increasing proximity of the desired location of the digital ultrasound image portion to a part of the digital ultrasound image representing a shortest distance to an ultrasound transducer.

11. The computer-implemented method of any one of claims 1 to 10, wherein the set of the one or more spatial filtering functions comprises a contrast enhancement function configured to enhance a contrast of the digital ultrasound image portion.

12. The computer-implemented method of any one of claims 1 to 11, further comprising displaying (230) the filtered digital ultrasound image portion at an output user interface.

13. A computer program product comprising computer program code means which, when executed on a computing device having a processing system, cause the processing system to perform all of the steps of the method according to any one of claims 1 to 12.

14. A processing system (120, 400) for processing a digital ultrasound image (310), the processing system being configured to:

receive (210) an indicator of at least one display parameter defining, as a digital ultrasound image portion, a portion of the digital ultrasound image for display; and
process (220) the digital ultrasound image portion using one or more spatial filtering functions (321, 322, 323) to produce a filtered digital ultrasound image portion (329), wherein one or more parameters of a set (321, 323) of the one or more spatial filtering functions are responsive to the indicator of the at least one display parameter.

**15.** An interface system comprising:

the processing system (120) of claim 14; and
an output user interface (140) configured to display the digital ultrasound image portion.

FIG. 1

FIG. 2

315

310

315

321 322 323

θ$_i$ θ$_j$

329

300

FIG. 3

FIG. 4

Europäisches
Patentamt

European
Patent Office

Office européen
des brevets

# EUROPEAN SEARCH REPORT

**Application Number**

EP 24 20 6607

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/348247 A1 (MCLAUGHLIN GLEN W [US] ET AL) 3 December 2015 (2015-12-03)<br>* figure 5 *<br>* paragraph [0001] *<br>* paragraph [0037] *<br>* paragraph [0054] *<br>* paragraph [0056] *<br>* paragraph [0076] - paragraph [0078] *<br>----- | 1-15 | INV.<br>A61B8/08<br>A61B8/00<br>G06T5/00<br>G06T5/70 |
| A | WO 2014/144278 A1 (SENO MEDICAL INSTR INC [US]) 18 September 2014 (2014-09-18)<br>* paragraph [0419] *<br>----- | 1-15 | |
| A | HUANG CHENGWU ET AL: "Debiasing-Based Noise Suppression for Ultrafast Ultrasound Microvessel Imaging",<br>IEEE TRANSACTIONS ON ULTRASONICS, FERROELECTRICS, AND FREQUENCY CONTROL,<br>IEEE, USA,<br>vol. 66, no. 8, 1 August 2019 (2019-08-01)<br>, pages 1281-1291, XP011737048,<br>ISSN: 0885-3010, DOI:<br>10.1109/TUFFC.2019.2918180<br>[retrieved on 2019-07-30]<br>* abstract *<br>----- | 1-15 | |
| A | US 2010/130855 A1 (LUNDBERG ANDREW K [US] ET AL) 27 May 2010 (2010-05-27)<br>* claim 1 *<br>----- | 1-15 | TECHNICAL FIELDS SEARCHED (IPC)<br><br>A61B<br>G06T |

The present search report has been drawn up for all claims

| Place of search | Date of completion of the search | Examiner |
|---|---|---|
| Munich | 5 March 2025 | Willig, Hendrik |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 20 6607

This annex lists the patent family members relating to the patent documents cited in  the above-mentioned European search report.
The members are as contained in the European Patent Office EDP file on
The European Patent Office is in no way liable for these particulars which are merely given for the purpose of information.

05-03-2025

| Patent document cited in search report | | | Publication date | Patent family member(s) | | Publication date |
|---|---|---|---|---|---|---|
| US 2015348247 | A1 | | 03-12-2015 | CN 106659468 A | | 10-05-2017 |
| | | | | CN 110533613 A | | 03-12-2019 |
| | | | | CN 110610466 A | | 24-12-2019 |
| | | | | US 2015348247 A1 | | 03-12-2015 |
| | | | | US 2017091915 A1 | | 30-03-2017 |
| | | | | US 2018197280 A1 | | 12-07-2018 |
| | | | | WO 2015184300 A1 | | 03-12-2015 |
| WO 2014144278 | A1 | | 18-09-2014 | NONE | | |
| US 2010130855 | A1 | | 27-05-2010 | US 2010130855 A1 | | 27-05-2010 |
| | | | | WO 2010059485 A1 | | 27-05-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KIM, SUNG** ; **RILEY CASPER**. *Applications of convolution in image processing with MATLAB*, 2013, 1-20

- **ROERDINK, JOS BTM**. An introduction to digital image processing. *Digital Image Analysis of Microbes: Imaging, Morphometry, Fluorometry and Motility Techniques and Applications*, 1998, vol. 1